# EUROPEAN PATENT APPLICATION

(11) **EP 3 257 864 A1**
(43) Date of publication of application: **20.12.2017**
(21) Application number: 16305734.2
(22) Date of filing: 16.06.2016
(51) Int. Cl.: C07K 14/575

(54) **METABOLICALLY STABLE SPEXIN PEPTIDE ANALOGS**

(71) Applicant: Université de Strasbourg, 67000 Strasbourg (FR); Centre National de la Recherche Scientifique CNRS, 75794 Paris Cedex 16 (FR)
(72) Inventor: BONNET, Dominique, 67118 Geispolsheim (FR); SIMONIN, Frédéric, 67190 Gresswiller (FR); LE COZ, Glenn-Marie, 67000 Strasbourg (FR); ESTEOULLE, Lucie, 67400 Illkirch-Graffenstaden (FR)
(74) Representative: Novagraaf Technologies

(57) **Abstract**

The present invention concerns metabolically stable and non-immunogen spexin analogs that are completely hydrosoluble at physiological pH, and their use for the prevention or the treatment of diseases mediated by the GALR2 receptor.

## Description

### Field of the invention

The present invention concerns metabolically stable and non-immunogen spexin analogs that are completely soluble at physiological pH, and their use for the prevention or the treatment of diseases mediated by the galanin receptor 2 (GALR2).

### Background of the invention

The galanin receptor 2 (GALR2), belonging to G-protein coupled receptors (GPCRs) responsible for transducing a signal within a cell, was isolated from rat hypothalamus extract (Howard et al., FEBS Letts., 405: 285-290, 1997; Smith et al., J. Biol. Chem. 272: 24612-24616, 1997; Wang et al., Mol. Pharmacol., 52: 337-343, 1997) [1-3]. This receptor couples to GI/Go, Gq/G11 or G12 G-protein types, which means that this subtype of galanin receptors can mediate stimulatory as well as inhibitory effects. The distribution of GALR2 is widespread within the CNS but different from that of GALR1. The dorsal root ganglia (DRG) expresses the highest level of GALR2 in the rat (O'Donnell et al., J. Comp. Neurol., 409: 469-481, 1999; Waters et Kraus, Neurosci., 95: 265-271, 2000) [4, 5], while low levels of GALR2 mRNA were detected in the rat locus coeruleus (LC) and in the dorsal raphe nucleus (DRN) region (O'Donnell et al., J. Comp. Neurol., 409: 469-481, 1999) [4]. The mouse GALR2 has been reported in the mouse brain but not in the DRN (Hawes et al., J. Comp. Neurol., 479: 410-423, 2004) [6]. Although GALR2 is known to display a function particularly in anxiety, depression, appetite regulation and pain modulation the further determination of its functions would benefit from a stable and selective agonist that acts only at GALR2.

Neuropeptide Q, alias Spexin, is a hormone recently discovered by Mirabeau et al. (Genome Res., 17: 320-327, 2007) [7]. The authors developed a Hidden Markov Model (HMM) based on algorithm searches that integrates several peptide hormone sequence features to identify novel peptide hormone. The predicted mature spexin peptide sequence of 14 amino acids flanked by dibasic cleavage sites is evolutionarily conserved across vertebrate species. Spexin expression in brain regions and peripheral tissues of various mammals suggests multiple physiological functions for spexin. Recently spexin was implicated in regulation of feeding behaviors and related metabolic processes (Walewski et al., Obesity 22 : 1643-1652, 2014) [11]. In addition, spexin is likely involved in reproduction, cardiovascular/renal function, and nociception (Liu et al., Mol. Cell Endocrinol., 374(1-2): 65-72, 2013 ; Toll et al., Faseb J., 26 : 947-954, 2012) [12, 13]. However the precise roles of spexin in these processes were not well understood due a lack of information on the spexin receptor.

More recently it has been found that spexin is an endogenous ligand that acts at GALR2 and GALR3 but not at GALR1 (Kim et al., Endocrinol., 155: 1864-1873, 2014) [8]. Based upon the GALR2/spexin interaction, it has been proposed to develop screening assays for agents modulating the activity of the GALR2 and diagnostic assays, as well as kits for performing the same (International Application WO 2012/042455) [9].

Considering the interaction between Spexin and galanin receptors, development of more stable and subtype-selective GALR2 agonists would lead to marked progress in treatment of galanin receptors-related diseases or disorders, in particular of GALR2-related diseases or GALR2-related disorders. Therefore it has been developed spexin-based human GALR2 specific agonists with increased stability in serum and anxiolytic effect in mice (Reyes-Alcaraz et al., Scientific Reports, 6: 21453, DOI: 10.1038/srep21453, February 24, 2016) [10].

### Summary of the invention

The present invention relates to novel metabolically stable fluoropeptides derived from the Neuropeptide Q, alias Spexin. Such spexin analogs are non-immunogen, completely hydrosoluble at physiological pH and have a better affinity toward GALR2 than the endogenous Neuropeptide Q they derived from. There is a therapeutic interest of using said metabolically stable analogs as therapeutic agents useful for the treatment of GALR2-related diseases or GALR2-disorders, for example central nervous System (CNS) disorders or cardiovascular disorders.

In one aspect, the present invention relates to a spexin analog having the following peptide of formula (I) :

Xaa1-Trp-Xaa2-Xaa3-Gln-Ala-Xaa4-Xaa5-Tyr-Leu-Lys-Gly-Xaa6-Xaa7 (I)

wherein said peptide of formula (I) (SEQ ID NO: 1) is covalently linked to a fluorocarbon group, directly or through a linker selected from the group consisting of a PEG or a peptide having from 1 to 6 amino acids, either on the alpha-amino or the epsilon-amino group of at least one lysine of the peptide formula (I), and when the spacer is a lysine, the fluorocarbon group is directly linked to the epsilon-amino group of said spacer, and wherein :
Xaa1 is Asn, Pro or Ala or hydrogen ;
Xaa2 is Thr or Pro ;
Xaa3 is Pro or Ala ;
Xaa4 is Met or Ala ;
Xaa5 is Leu or Ala ;
Xaa6 is Ala, Pro or NH2 ;
Xaa7 is Gin, Pro, Ala or NH2, when Xaa6 is not NH2.

Said spexin analog is advantageously metabolically stable, non-immunogen analog, and completely hydrosoluble at physiological pH.

As used herein, the expression "completely hydrosoluble at physiological pH" means that the fluoropeptides of the present invention as above described have at least 50% hydrophobic amino acid residues and have an overall positive net charges and a final solubility in aqueous mixture above 100 µM by visual inspection of the cloudiness of the resulting dispersion and/or solubility measurements by classical physicochemical methods.

As used herein, the expression "non-immunogen" means that the fluoropeptides of the present invention as above described are not derived from any antigens capable of inducing immune response in an animal, including humans. Antigens may be derived from a virus, bacterium or mycobacterium, parasite, fungus, or any infectious agent or an autologous antigen or allergen. The fluoropeptides described in the invention are not included in any vaccine.

As used herein, the expression "metabolically stable" means that the fluoropeptides of the present invention as described above, have at least the half-life of the native peptide, and in particular when linked to a charged linker, have a half-life at twice longer than native peptide, or at least 20 min or more than one hour, as measured in the stability assay performed in human plasma.

As used herein, the term "fluorocarbon" includes either, perfluorocarbon (where all hydrogen are replaced by fluor) or, hydrofluorocarbon (which contains both C-H and C-F bonds).

The fluorocarbon group may comprise one or more chains derived from perfluorocarbon or mixed fluorocarbon/hydrocarbon radicals, and may be saturated or unsaturated, each chain having from 3 to 30 carbon atoms. The fluorocarbon group is linked to the peptide through a covalent linkage, for example via NH₂-group of a lysine of the peptide of formula (I). The coupling to the peptide may be achieved through a functional group for linkage to -NH₂, being naturally present on the lysine of the peptide of formula I, or onto a linker. Modify the nature of the linkage between the fluorocarbon chain and the peptide allows modulating the stability and/or solubility of the peptide. Examples of such linkages between the fluorocarbon chain and the peptide of formula I include amide, hydrazine, disulphide, thioether, ester and oxime bonds.

Optionally, a cleavable linker element (peptide or non-peptidic) may be incorporated to permit cleavage of the peptide of formula I from the fluorocarbon group. The linker may also be incorporated to assist in the synthesis of the fluoropeptide and to improve its stability and/or solubility, for example by including additional charges. So charged linker may be particularly useful especially if the peptide to which it is linked has no cationic aminoacids (*i.e.* lysine, histidine, arginine) at its N-terminal end. Examples of linkers include polyethylene glycol (PEG), or a peptide having about 1 to 6 amino acids, natural on non-natural ones, that may be cleaved by proteolytic enzymes or not. Preferably said amino acids are chosen from the group consisting of basic or aliphatic amino acids, more preferably from histidine, lysine, arginine and glycine. For example, the linker may be Arg-Gly-Arg.

Thus, the fluorocarbon group of the spexin analog of the present invention has chemical formula (II) CₘFₙ-CyHₓ(L) wherein m=3 to 30, n<2m+1, y=0 to 2, x≤2y, (m+y)=3 to 30, and L, which is optional, is a functional group leading to covalent attachment to the peptide. For example said functional group is a carbonyle -C(O)- that forms an amide bond with the -NH2 of a lysine.

According to a particular embodiment of the above formula II of the fluorocarbon, m=5 to 15, preferably m=5 to 15 and y=1 to 4. According to another particular embodiment, the formula of the fluorocarbon group is perfluoroundecanoic acid of formula (A) 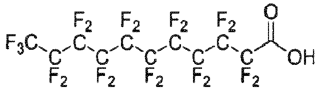 or alternatively is 2H,2H,2H,3H,3H-perfluoroundecanoic acid of formula (B) 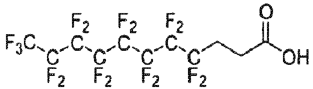 or alternatively is heptadecafluoro-pentadecanoic acid of formula (C)

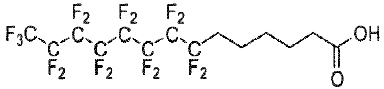

In these cases it is to be noted that reducing the length of the fluorocarbon group of formula (II), for example by deleting at least two CF₂ groups, preferably at least four CF₂ groups, can increase the solubility and/or plasmatic stability of the peptide of formula (I) to which it is linked.

According to a particular embodiment, the spexin analog of formula (I) as above defined is further covalently linked to an acetyl group and/or an acyl group -C(O)R where R is a C₇₋₃₀ alkyl. For example it has the following formula (III) CH₃-C_{y}Hx-C(O)- wherein y=7 to 30, preferably y=10 to 20, more preferably y=14, and x=2y.

Said fluorocarbon group or further acetyl and:or acyl group can be linked at the N-terminal part of the peptide directly through a lysine, either on the alpha-amino or the epsilon-amino groups.

According to a particular embodiment of a fluoropeptide of formula (I), the present invention relates to a spexin analog selected from the group consisting of :
i) CF₃(CF₂)₇(CH₂)₂C(O)-Asn-Trp-Thr-Pro-Gln-Ala-Met-Leu-Tyr-Leu-Lys-Gly-Ala-Gln (compound LE144; SEQ ID NO: 2);
ii) CF₃(CF₂)₇(CH₂)₂C(O)-Arg-Gly-Arg-Asn-Trp-Thr-Pro-Gln-Ala-Met-Leu-Tyr-Leu-Lys-Gly-Ala-Gln (compound LE130; SEQ ID NO: 3);
iii) Acetyl-Asn-Trp-Thr-Pro-Gln-Ala-Met-Leu-Tyr-Leu-Lys-Gly-Ala-Gln-Lys(C(O)(CH₂)₂(CF₂)₇CF₃) (compound LE128; SEQ ID NO: 4);
iv) Acetyl-Asn-Trp-Thr-Pro-Gln-Ala-Met-Leu-Tyr-Leu-Lys-Gly-Ala-Gln-Lys(Arg-Gly-Arg-(C(O)(CH₂)₂(CF₂)₇CF₃)) (compound LE146; SEQ ID NO: 5);
v) a spexin analog with an amino acid sequence having at least 80% identity with the sequence of (i), (ii), (ii) or (iv).

In another aspect, the present invention also relates to a pharmaceutical composition comprising a spexin analog of formula (I) as above described, and one or more pharmaceutically acceptable excipient.

Such pharmaceutical composition is prepared for administration to a subject in need thereof and which include therapeutically effective amount of one or more of the metabolically stable spexin analog(s) of the present invention. The therapeutically effective amount of a metabolically stable spexin analog will depend on the route of administration, the type of mammal that is the subject and the physical characteristics of the subject being treated. Specific factors that can be taken into account include disease severity and stage, weight, diet and concurrent medication. The relationship of these factors to determining a therapeutically effective amount of the disclosed compounds is well known and understood by those of ordinary skill in the art. Pharmaceutically acceptable excipients or vehicles are also well known to those of ordinary skill in the art. Standard excipients include solutions such as sterile water, saline, and buffered solutions at physiological pH. For example, pharmaceutical excipients include thickeners, diluents, buffers, preservatives, surface active agents and the like in addition to the spexin analog of choice. The pharmaceutically composition can be prepared in the form of aqueous solutions, lyophilized or other dried formulations (powder, granules, lozenges, etc...). In general the nature of the excipient or vehicle will depend on the particular mode of administration being employed. The pharmaceutically composition can be administered intravenously, intramuscularly, subcutaneously, intracerebroventricularly, orally, intraperitoneally, etc... or in an aerosol form.

The dosing is selected by those of ordinary skill in the art so that a therapeutically effect is achieved, and depends on the route of administration and the dosage form that is used. Total daily dose of a peptide administered to a subject in single or divided doses may be in amounts, for example, of from about 0.001 to about 100mg/kg body weight daily, preferably 0.01 to 10mg/kg/day. Dosage unit compositions may contain such amounts of such submultiples thereof as may be used to make up the daily dose. It will be understood, however, that the specific dose level for any particular patient will depend upon a variety of factors including the body weight, general health, sex, diet, time and route of administration, rates of absorption and excretion, combination with other drugs and the severity of the particular disease being treated.

In another aspect, the present invention also relates to a spexin analog of the present invention, for use as a drug.

In another aspect, the present invention also relates to a spexin analog of the present invention, for use in the prevention or treatment of a GALR2-related disease or GALR2-related disorder, preferably selected from central nervous system (CNS) disorders, cardiovascular disorders, nociception, renal disorders, neuroinflammation, etc.... For example said diseases or disorders include, but are not limited to:
- cardiovascular disease: heart failure, kidney diseases (*e.g.* renal failure, nephritis, etc...), hypertension, pulmonary hypertension, cirrhosis, arteriosclerosis, pulmonary emphysema, pulmonary oedema, stroke, brain ischemia, myocardial impairment in sepsis, cardiomyopathy;
- the syndrome of inappropriate antidiuretic hormone (SIADH);
- metabolic diseases: obesity, anorexia, hyperphagia, polyphagia, hypercholesterolemia, hyperglyceridemia, hyperlipemia;
- various types of dementia: senile dementia, Alzheimer's disease, cerebrovascular dementia, dementia due to genealogical denaturation degenerative disesases, dementia resulting from infectious diseases, dementia associated with endocrine diseases, metabolic diseases, or poisoning, dementia caused by tumors, and dementia due to traumatic diseases, depression, hyperactive child syndrome, disturbance of consciousness, anxiety disorder, schizophrenia, phobia;
- pain and hyperalgesia.

The present invention will be further illustrated by the following figures and examples. However these examples and figures should not be interpreted in any way as limiting the scope of the present invention.

### Brief description of the drawings

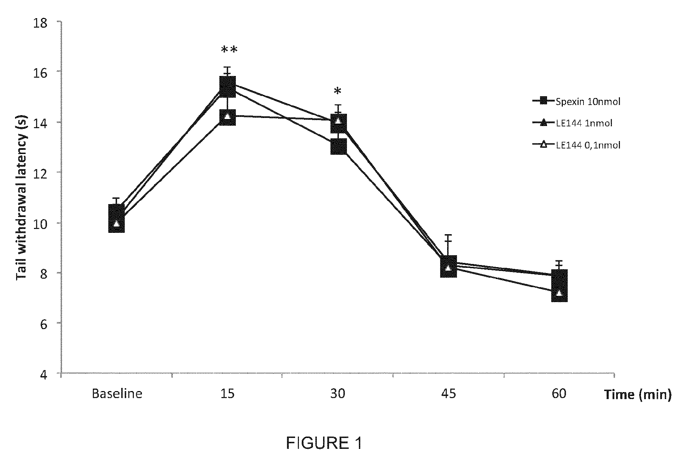

Figure 1 represents the *in vivo* antinociceptive activity of spexin and analog LE144 in mice. For the statistical analysis, one way Anova with Dunett post hoc test was used; *p<0,05; **p<0,01 and each time point was evaluated regarding the corresponding baseline for each group.

### EXAMPLES

### EXAMPLE 1: GENERAL MATERIAL AND METHODS

Reagents were obtained from commercial source and used without any further purification. Fmoc-L-amino acids were purchased from Novabiochem, Polypeptides and Iris Biotech. Fmoc-protected Rink Amide NovaGel® resin was purchased from Novabiochem and the overall yields for the solid-phase syntheses were calculated based on the initial loadings provided by the supplier (0.7 mmol/g). Fmoc-protected Wang NovaGel® resin was purchased from Novabiochem and the overall yields for the solid-phase syntheses were calculated based on the initial loadings provided by the supplier (0.1 mmol/g).

### Analytical reverse-phase high performance liquid chromatography (RP-HPLC) analysis

Analysis were performed either on a C18 Sunfire column (5µm, 4.6mm x 150mm) using a linear gradient (5% to 95% in 20min, flow rate of 1mL.min⁻¹) of solvent B (0.1% TFA in CH₃CN, v/v) in solvent A (0.1% TFA in H₂O, v/v). Detection was set AT 220nm and 254nM.

### Semi-preparative RP-HPLC chromatography purifications

Purifications were performed on Sunfire C18 column (5µm, 19 x 150mm) on Gilson PLC2020 with absorption detection. The separation was achieved using successive isocratic and linear gradients (5min at 5%; 5% to 60% in 30min; 60% to 100% in 10min; flow rate of 20mL.min⁻¹) of solvent B (0.1% TFA in CH₃CN, v/v) in solvent A (0.1% TFA in H₂O, v/v).

### Liquid chromatography mass spectra (LC-MS) analysis

Analysis were obtained on a ZQ (Z quadripole) Waters/Micromass spectrometer equipped with an X-Terra C18 column (0.5µm, 4.6mm x 50mm) using electrospray ionization mode (ESI).

### High resolution mass spectra (HR-MS) analysis

Analysis were acquired on a Bruker MicroTof mass spectrometer, using electrospray ionization (ESI) and a time-of-flight analyzer (TOF) or on an Autoflex II TOF/TOF Bruker mass spectrometer using matrix-assisted laser desorption/ionization technique (MALDI) and a time-of-light analyzer (TOF).

### General protocol for standard automated solid-phase peptide synthesis (SPPS)

Standard automated solid-phase peptide synthesis (SPPS) were performed on an Applied Biosystem ABI 433A synthesizer (Appelar, France). The elongation was carried out by coupling a 10-fold excess of Fmoc-L-amino acid derivatives, using 2-(1H-benzotriazol-1-yl)-1,1,3,3-tetramethyluronium hexafluorophosphate (HBTU), 1-hydroxybenzotriazole (HOBt), and diisopropylethylamine (Hünig's base) (DIPEA) as coupling reagents in N,N-dimethylformamide (DMF) as solvent. At the end of the peptide sequence synthesis, the resin was washed with CH₂Cl₂ and MeOH and then dried *in vacuo*. After each coupling step, Fmoc deprotection was performed by treatment with piperidine monitored by UV at 301 nm.

### General protocol for peptide elongation by manual SPPS

Peptide elongation was performed starting from fried resin previously synthetized by standard automated SPPS. Non-automated SPPS were performed in polypropylene tubes equipped with polyethylene frits and polypropylene caps using an orbital agitator shaking device.

The Fmoc-protected resin (1 equiv) was swollen 1h in DMF and the excess solvent was removed by filtration. Cleavage of the Fmoc protecting group was performed in a solution of 20% (v/v) piperidine in DMF (2 times for 15min). The piperidine solution was drained off and the resin was washed with successively DMF, CH₂Cl₂ and MeOH (3x0.5mL).

All Fmoc-protected amino acids (4 equiv) were coupled in N,N-dimethylformamide (DMF) for 45min using HBTU (3.8 equiv) and HOBt (4 equiv) with N,N-diisopropylethylamine (DIEA) (12 equiv) as activating agents. The excess solvent was removed by filtration and the resin was washed with successively DMF, CH₂Cl₂ and MeOH (3x0.5mL).

The cycle of coupling, washing and deprotection were repeated until the targeted peptides were obtained. The completion of couplings and Fmoc deprotections were monitored with ninhydrin test and TNBS test:
- Ninhydrin test (for primary amines) : Resin beads were suspended in 2 drops of a solution containing 5g of ninhydrin dissolved in 100mL of ethanol, 2 drops of a solution containing 80g of liquefied phenol in 20mL of ethanol, and 2 drops of a 0.001 M aqueous solution of potassium cyanide to 98mL pyridine. The mixture was heated at 100°C for 1min. The color positive test (presence of free amino groups). A yellow or blue solution and yellow beads indicate a negative test.
- TNBS test (for primary amines): Resin beads were suspended in 2 drops of a solution containing 10% (v/v) DIPEA in DMF and 2 drops of a solution containing 2,4,6-trinitrobenzenesulfonic acid (TNBS) in DMS. The color of the solution and the beads were observed. A yellow-red solution and red beads indicate a positive test. A yellow-red solution and yellow beads indicate a negative test.

### General protocol for peptide elongation with a perfluoroalkyl chain

The resin containing the peptide sequence of interest (1 equiv) was swollen in DMF, and the excess solvent was removed by filtration. A solution of piperidine in DMF (20% v/v - 0.5mL) was added, and the mixture was shaken at room temperature for 15min. The solution was drained, and the operation was repeated for 15min. The solution was drained, and the resin was washed with DMF and CH₂Cl₂. In a separate vial, DIEA (8 equiv) was added to a solution of 4,4,5,5,6,6,7,7,8,8,9,9,10,10,11,11-heptadecafluoroundecanoic acid (2 equiv), HBTU (2 equiv), and HOBt (1.9 equiv) in DMF (0.5mL). The mixture was stirred at room temperature for 1 min and was added to the resin. The mixture was shaken at room temperature for 2h. The solution was drained and the resin was washed with DMF, CH₂Cl₂, and MeOH the dried *in vacuo*.

### General protocol for resin cleavage

The dried resin was treated with TFA/Phenol/Thioanisole/1,2-Ethanedithiol/water (10mL/0.75g/0.5mL/0.25mL/0.5mL) and the mixture was shaken at room temperature for 3h. The filtrate was collected in a cold diethyl ether solution and the beads washed with TFA. The solution was centrifuged at 3000rpm for 2min. The precipitate was washed in a cold diethyl ether solution and centrifuged at 3000rpm for 2 min. The diethyl ether solution was eliminated and the precipitate was dried *in vacuo*. The crude product was purified by semi-preparative RP-HPLC and lyophilized.

### EXAMPLE 2: SPEXIN ANALOGS SYNTHESIZED AND THEIR CHARACTERIZATION

### Spexin analogs synthesis

LE-144

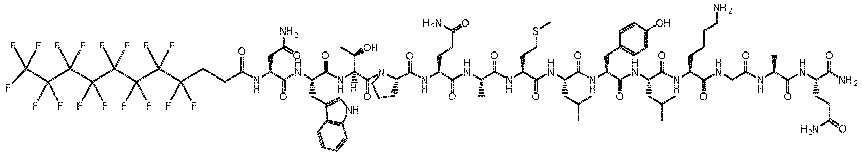

Fmoc-Asn-Trp-Thr-Pro-Gln-Ala-Met-Leu-Tyr-Leu-Lys-Gly-Ala-Gln-Rink resin (23µmol), 4,4,5,5,6,6,7,7,8,8,9,9,10,10,11,11-heptadecafluoroundecanoic acid (2 equiv), HBTU (2 equiv), HOBt (1.9 equiv) and DIEA (8 equiv) were reacted according the general procedure, affording the title compound (6.9 mg, 14%) as a white solid. t_{R} =13.60 min (>95% purity at 220 nm); HRMS (ESI) calcd for C₈₅H₁₁₇F₁₇N₂₀O₂₀S: 2092.82023; found: 2092.81872.

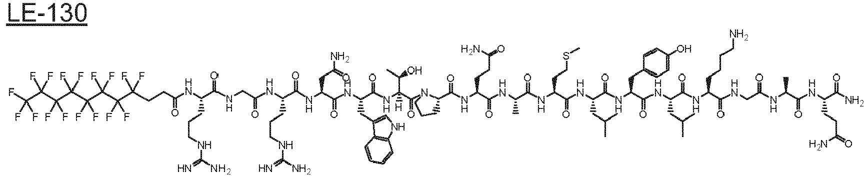

Fmoc-Asn-Trp-Thr-Pro-Gln-Ala-Met-Leu-Tyr-Leu-Lys-Gly-Ala-Gln-Rink resin (29 µmol), Fmoc-Arg(Pbf)-OH (4 equiv), HBTU (3.8 equiv), HOBt (4 equiv) and DIEA (12 equiv) were reacted according the general procedure. Fmoc-Gly-OH (4 equiv) and Fmoc-Arg(Pbf)-OH (4 equiv) were then added with the same procedure. Finally, 4,4,5,5,6,6,7,7,8,8,9,9,10,10,11,11-heptadecafluoroundecanoic acid (2 equiv), HBTU (2 equiv), HOBt (1.9 equiv) and DIEA (8 equiv) were reacted according the general procedure, affording the title compound (10.5 mg, 13%) as a white solid. t_{R} =11.02 min (>95% purity at 220,8 nm); HRMS (ESI) calcd for C₉₉H₁₄₄F₁₇N₂₉O₂₃S: 2462.04391; found: 2462.05017.

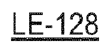

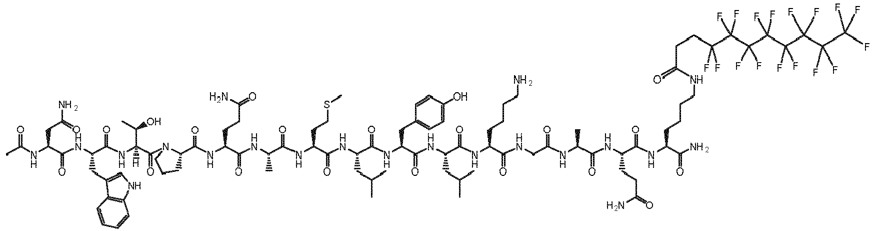

Fmoc-Asn-Trp-Thr-Pro-Gln-Ala-Met-Leu-Tyr-Leu-Lys-Gly-Ala-Gln-Lys(ivDde)-Rink resin (22 µmol) was treated in a solution of 20% (v/v) piperidine in DMF (2 times for 15 min). The piperidine solution was drained off and the resin was washed with successively DMF, CH₂Cl₂ and MeOH (3x0.5 mL). Acetylation of NH₂-Asn-Trp-Thr-Pro-Gln-Ala-Met-Leu-Tyr-Leu-Lys-Gly-Ala-Gln-Lys(ivDde)-Rink resin was performed by adding 0.5 mL of a solution of 10% anhydride acetic and 5% DIEA in CH₂Cl₂ for 10 min. The acetylation solution was drained off and the resin was washed with 0.5 mL of CH₂Cl₂ for 2 min (3 times). Removal of the ivDde protecting group was performed in a solution of 2% (v/v) hydrazine in DMF (3 times for 3 min). The hydrazine solution was drained off and the resin was washed with successively DMF, CH₂Cl₂ and MeOH (3x0.5 mL). Finally, 4,4,5,5,6,6,7,7,8,8,9,9,10,10,11,11-heptadecafluoroundecanoic acid (2 equiv), HBTU (2 equiv), HOBt (1.9 equiv) and DIEA (8 equiv) were reacted according the general procedure. Following treatment in acidic conditions and precipitation, the crude mixture was purified by RP-HPLC, concentrated under vaccum and freeze-dried, affording the title compound (4.5 mg, 8.6%) as a white solid. t_{R} =13.11 min (>95% purity at 220 nm); HRMS (ESI) calcd for C₉₃H₁₃₁F₁₇N₂₂O₂₂S: 2262.92709; found: 2262.92680.

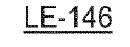

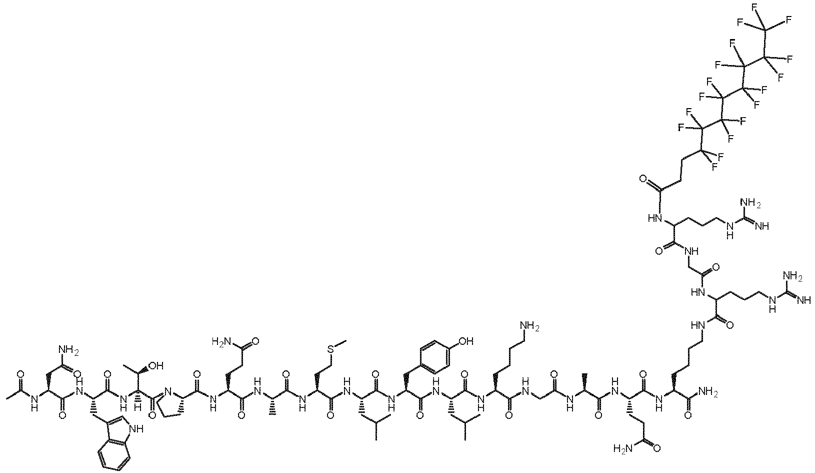

Fmoc protecting group of Fmoc-Asn-Trp-Thr-Pro-Gln-Ala-Met-Leu-Tyr-Leu-Lys-Gly-Ala-Gln-Lys(ivDde)-Rink resin (21 µmol) was performed in a solution of 20% (v/v) piperidine in DMF (2 times for 15 min). The piperidine solution was drained off and the resin was washed with successively DMF, CH₂Cl₂ and MeOH (3×0.5 mL). Acetylation of NH₂-Asn-Trp-Thr-Pro-Gln-Ala-Met-Leu-Tyr-Leu-Lys-Gly-Ala-Gln-Lys(CO(CH₂)₂C₈F₁₇)-Rink resin was performed by adding 0.5 mL of a solution of 10% anhydride acetic and 5% DIEA in CH₂Cl₂ for 10 min. The acetylation solution was drained off and the resin was washed with 0.5 mL of CH₂Cl₂ for 2 min (three times). Removal of the ivDde protecting group was performed in a solution of 2% (v/v) hydrazine in DMF (3 times for 3 min). The hydrazine solution was drained off and the resin was washed with successively DMF, CH₂Cl₂ and MeOH (3×0.5 mL). Fmoc-Arg(Pbf)-OH, Fmoc-Gly-OH and Fmoc-Arg(Pbf)-OH were successively reacted according the general procedure. N-Fmoc removal was followed by the coupling of 4,4,5,5,6,6,7,7,8,8,9,9,10,10,11,11-heptadecafluoroundecanoic acid according the general procedure. Following treatment in acidic conditions and precipitation, the crude mixture was purified by RP-HPLC, concentrated under vaccum and freeze-dried affording the title compound (3.1 mg, 5.6%) as a white solid. = 11.10 min (>95% purity at 220 nm); LCMS calcd for C₁₀₇H₁₅₈F₁₇N₃₁O₂₅S: 2632.15; found: 2633.17.

### Spexin analogs characterization

### Affinity

Evaluation of the affinity of compounds for the human galanin GAL2 receptor in transfected CHO cells determined in a radioligand binding assay: Experimental protocol : Cells membrane homogenates (4 µg protein) are incubated for 120 min at 22°C with 0.05 nM [¹²⁵I]galanin in the absence or presence of the test compound in a buffer containing 25 mM Tris-HCl (pH 7.4), 10 mM MgCl2 and 0.5% BSA. Nonspecific binding is determined in the presence of 1 µM porcine galanin. Following incubation, the samples are filtered rapidly under vacuum through glass fiber filters (GF/B, Packard) presoaked with 0.3% PEI and rinsed several times with ice-cold 50 mM Tris-HCl using a 96-sample cell harvester (Unifilter, Packard). The filters are dried then counted for radioactivity in a scintillation counter (Topcount, Packard) using a scintillation cocktail (Microscint 0, Packard). The results are expressed as a percent inhibition of the control radioligand specific binding. The standard reference compound is porcine galanin, which is tested in each experiment at several concentrations to obtain a competition curve from which its IC50 is calculated.

### EC50

Calcium mobilization: CHO cells expressing GalR2 were loaded with 2,5µM of Fluo-4 AM in the presence of 2.5 mM probenicid. Agonist-evoked increases in intracellular calcium were recorded over time (5 sec intervals over 220 sec) at 37 °C by using a Flexstation III (Molecular Devices, Sunnyvale, CA, USA). Fluorescence signals were recorded at 520 nm (excitation at 485 nm). Peak amplitudes were normalized to baseline and maximal fluorescence level elicited by 20 µM digitonin, and EC50 were calculated with Graphpad/Prism software.

### Solubility

The solubility of each spexin analogs was evaluated after dissolution in water to reach 100µM. The resulting solution was vortexed 1min following by 1 min in bath sonication. Solubility was then assessed by visual observation of the resulting dispersion (Clear/Cloudy and presence of particulates).

### Human plasma stability

This procedure is designed to determine the stability of a test compound in blood or plasma from human or animal species in a 96-well plate format. The test compound is quantified at 5 time points by HPLC-MS/MS analysis. Test concentration: 1 µM with a final DMSO concentration of 0.5 %. Experimental protocol : Blood or plasma are pre-warmed at 37 °C water bath for 5 min, followed by addition of the test compound. The incubation is performed in a 37 °C water bath for 2 h. An aliquot of the incubation mixture is transferred to acetonitrile at 0, 0.5, 1, 1.5 and 2 h, respectively. Samples are then mixed and centrifuged. Supernatants are used for HPLC-MS/MS analysis. Reference compounds Propoxycaine and propantheline are tested simultaneously with the test compound in each assay. Analytical methods Samples are analyzed by HPLC-MS/MS using selected reaction monitoring. The HPLC system consists of a binary LC pump with autosampler, a C-18 column, and a gradient. Conditions may be adjusted as necessary. Data analysis Peak areas corresponding to the test compound are recorded. The compound remaining (%) is calculated by comparing the peak area at each time point to time zero. The half-life is calculated from the slope of the initial linear range of the logarithmic curve of compound remaining (%) vs. time, assuming first order kinetics.

The results are presented in table 1 below:

**Table 1**

| **Peptide** | **Affinity % of inhibition at 100 nM** | **EC50 Ca²⁺ production (nM)** | **Solubility in water (µm)** | **Human plasma stability (t_{1/2}**, **min)** |
|---|---|---|---|---|
| **SPEXIN** | 68.3% | 37 ± 18 | >100 | 151 |
| **LE-144** | 100.0 % | 0.038 ± 0.008 | >100 | 150 |
| **LE-130** | 98.4 % | 0.089 ± 0.04 | >100 | 342 |
| **LE-128** | - | 2.3 ± 4.0 | - | - |
| **LE-146** | - | 8.4 ± 0.7 | - | - |

The results show that the spexin analogs tested exhibit a better affinity and efficiency than the native peptide (spexin), and at least the same or better hydrosolubility and/or plasma stability.

### EXAMPLE 3: IN VIVO RESULTS OBTAINED WITH SPEXIN ANALOGS

**Tail immersion test:** Nociception tests were performed on male, awake C57BL/6N male mice (25-30g weight; Janvier Labs, France). Animals were housed in groups of five per cage and kept under a 12h/12 h light/dark cycle at 21 ± 1°C with *ad libitum* access to food and water. Experiments were performed during the light-on phase of the cycle. Mice were habituated to the testing room and equipment and handled for 1 week before starting behavioural experiments. The nociceptive thermal threshold of mice was determined using the tail immersion test. Mice were restrained in a grid pocket and their tail was immersed in a thermostated water bath. The latency (in sec) for tail withdrawal from hot water (47 ± 0.5 °C) was taken as a measure of the nociceptive response. In the absence of any nociceptive reaction, a cut-off value of 25 sec was set to avoid tissue damage.

The results are shown in figure 1.

The results show that intracerebroventricular (icv) injection of 10nmol of spexin elicited an analgesic effect at 15 and 30 min after injection as revealed by a significant increase in tail immersion withdrawal latencies of the animals compared to baseline threshold (from 10,45 ± 0,53 sec basal threshold to 15,38 ± 0,57 sec at 15min and 13,06 ±0,87 sec at 30min; p<0,01 and p<0,05, respectively).

Icv injection of 1nmol and 0,1nmol of LE-144 evoked the same statistically significant analgesia after 15min and 30min (1nmol ; baseline : 10,12 ± 0,59sec, 15min : 15,58 ±0,61sec, 30min : 13,94 ±0,75 sec; 0,1nmol ; baseline : 9,99 ±0,5 sec, 15min : 14,26 ± 0,93 sec, 30min : 14,06 ± 0,34 sec).

### Reference listing

- 1.: Howard et al., FEBS Letts., 405: 285-290, 1997
- 2.: Smith et al., J. Biol. Chem. 272: 24612-24616, 1997
- 3.: Wang et al., Mol. Pharmacol., 52: 337-343, 1997
- 4.: O'Donnell et al., J. Comp. Neurol., 409: 469-481, 1999
- 5.: Waters et Kraus, Neurosci., 95: 265-271, 2000
- 6.: Hawes et al., J. Comp. Neurol., 479: 410-423, 2004
- 7.: Mirabeau et al., Genome Res., 17: 320-327, 2007
- 8.: Kim et al., Endocrinol., 155: 1864-1873, 2014
- 9.: International Application WO 2012/042455
- 10.: Reyes-Alcaraz et al., Scientific Reports, 6: 21453, DOI: 10.1038/srep21453, 2016
- 11.: Walewski et al., Obesity, 22 : 1643-1652, 2014
- 12.: Liu et al., Mol. Cell Endocrinol., 374(1-2): 65-72, 2013
- 13.: Toll et al., Faseb J., 26 : 947-954, 2012

## Claims

1. A spexin analog having the following peptide of formula (I) :
Xaa1-Trp-Xaa2-Xaa3-Gln-Ala-Xaa4-Xaa5-Tyr-Leu-Lys-Gly-Xaa6-Xaa7 (I)
wherein said peptide of formula (I) is covalently linked to a fluorocarbon group, directly or through a linker selected from the group consisting of a PEG or a peptide having from 1 to 6 amino acids, either on the alpha-amino or the epsilon-amino group of at least one lysine of the peptide formula (I), and when the spacer is a lysine, the fluorocarbon group is directly linked to the epsilon-amino group of said spacer, and wherein :
Xaa1 is Asn, Pro or Ala or hydrogen ;
Xaa2 is Thr or Pro ;
Xaa3 is Pro or Ala ;
Xaa4 is Met or Ala ;
Xaa5 is Leu or Ala ;
Xaa6 is Ala, Pro or NH2 ;
Xaa7 is Gin, Pro, Ala or NH2, when Xaa6 is not NH2.

2. The spexin analog according to claim 1, wherein said peptide is further covalently linked to an acetyl group and/or an acyl group -C(O)R where R is a C₇₋₃₀alkyl.

3. The spexin analog according to either one of claim 1 or 2, which is a metabolically stable and non-immunogen analog that is completely hydrosoluble at the physiological pH.

4. The spexin analog according to any one of claims 1 to 3, wherein said fluorocarbon group linked to said peptide of formula (I) has the following formula (II):
CₘFₙ-C_{y}Hₓ(L) (II)
wherein m=3 to 30, n≤2m+1, y=0 to 2, x≤2y, (m+y)=3 to 30, and L, which is optional, is a linker selected from the group consisting of a PEG or a peptide having from 1 to 6 amino acids.

5. The spexin analog according to any one of claims 1 to 4, wherein said acyl group has the following formula (III):
CH₃-C_{y}Hₓ-C(O)- (III)
wherein y=7 to 30, x=2y.

6. The spexin analog according to any one of claims 1 to 5, which is selected from:
vi) CF₃(CF₂)₇(CH₂)₂C(O)-Asn-Trp-Thr-Pro-Gln-Ala-Met-Leu-Tyr-Leu-Lys-Gly-Ala-Gln;
vii) CF₃(CF₂)₇(CH₂)₂C(O)-Arg-Gly-Arg-Asn-Trp-Thr-Pro-Gln-Ala-Met-Leu-Tyr-Leu-Lys-Gly-Ala-Gln;
viii) Acetyl-Asn-Trp-Thr-Pro-Gln-Ala-Met-Leu-Tyr-Leu-Lys-Gly-Ala-Gln-Lys(C(O)(CH₂)₂(CF₂)₇CF₃);
ix) Acetyl-Asn-Trp-Thr-Pro-Gln-Ala-Met-Leu-Tyr-Leu-Lys-Gly-Ala-Gln-Lys(Arg-Gly-Arg-(C(O)(CH₂)₂(CF₂)₇CF₃));
x) a spexin analog with an amino acid sequence having at least 80% identity with the sequence of (i), (ii), (ii) or (iv).

7. The spexin analog according to any one of claims 1 to 6, for use as a drug.

8. The spexin analog according to any one of claims 1 to 6, for use in the treatment of a GALR2-related disease or a GALR2-related disorder selected from :
- cardiovascular disease: heart failure, kidney diseases (e.g. renal failure, nephritis, etc...), hypertension, pulmonary hypertension, cirrhosis, arteriosclerosis, pulmonary emphysema, pulmonary oedema, stroke, brain ischemia, myocardial impairment in sepsis, cardiomyopathy;
- the syndrome of inappropriate antidiuretic hormone (SIADH);
- metabolic diseases: obesity, anorexia, hyperphagia, polyphagia, hypercholesterolemia, hyperglyceridemia, hyperlipemia;
- various types of dementia: senile dementia, cerebrovascular dementia, dementia due to genealogical denaturation degenerative disesases, dementia resulting from infectious diseases, dementia associated with endocrine diseases, metabolic diseases, or poisoning, dementia caused by tumors, and dementia due to traumatic diseases, depression, hyperactive child syndrome, disturbance of consciousness, anxiety disorder, schizophrenia, phobia;
- pain and hyperalgesia.

9. A pharmaceutical composition comprising a spexin analog according to any one of claims 1 to 6, and one or more pharmaceutically acceptable excipient.


SEQUENCE LISTING

<110> UNIVERSITE DE STRASBOURG
CNRS
BONNET, Dominique
SIMONIN, Frédéric
LE COZ, Glenn-Marie
ESTEOULLE, Lucie

<120> METABOLICALLY STABLE SPEXIN ANALOGS

<130> BNT220810EP00

<160> 5

<170> PatentIn version 3.5

<210> 1
<211> 14
<212> PRT
<213> Artificial Sequence

<220>
<223> spexin analog of formula (I)

<220>
<221> MISC_FEATURE
<222> (1)..(1)
<223> Xaa is Asn, Pro , Ala or hydrogen

<220>
<221> MISC_FEATURE
<222> (3)..(3)
<223> Xaa is Thr or Pro

<220>
<221> MISC_FEATURE
<222> (4)..(4)
<223> Xaa is Pro or Ala

<220>
<221> MISC_FEATURE
<222> (7)..(7)
<223> Xaa is Met or Ala

<220>
<221> MISC_FEATURE
<222> (8)..(8)
<223> Xaa is Leu or Ala

<220>
<221> MISC_FEATURE
<222> (13)..(13)
<223> Xaa is Ala, Pro or NH2

<220>
<221> MISC_FEATURE
<222> (14)..(14)
<223> Xaa is Gln, Pro, Ala or NH2, when Xaa at position 13 is not NH2

<400> 1

Xaa Trp Xaa Xaa Gln Ala Xaa Xaa Tyr Leu Lys Gly Xaa Xaa
1               5                   10

<210>  2
<211>  14
<212>  PRT
<213>  Artificial Sequence

<220>
<223>  compound LE144

<400>  2

Asn Trp Thr Pro Gln Ala Met Leu Tyr Leu Lys Gly Ala Gln
1               5                   10

<210>  3
<211>  17
<212>  PRT
<213>  Artificial Sequence

<220>
<223>  Compound LE 130

<400>  3

Arg Gly Arg Asn Trp Thr Pro Gln Ala Met Leu Tyr Leu Lys Gly Ala
1               5                   10                  15

Gln

<210>  4
<211>  15
<212>  PRT
<213>  Artificial Sequence

<220>
<223>  Compound LE 128

<400>  4

Asn Trp Thr Pro Gln Ala Met Leu Tyr Leu Lys Gly Ala Gln Lys
1               5                   10                  15

<210>  5
<211>  18
<212>  PRT
<213>  Artificial Sequence

<220>
<223>  Compound LE 146

<400>  5

Asn Trp Thr Pro Gln Ala Met Leu Tyr Leu Lys Gly Ala Gln Lys Arg
1               5                   10                  15

Gly Arg